(19) Europäisches Patentamt
European Patent Office
Office européen des brevets

(11) Publication number : **0 399 606 B1**

(12) # EUROPEAN PATENT SPECIFICATION

(45) Date of publication of patent specification : **26.10.94 Bulletin 94/43**

(51) Int. Cl.$^5$ : **H04N 5/32**, A61B 6/00

(21) Application number : **90201274.9**

(22) Date of filing : **21.05.90**

(54) **X-ray exposure synchronisation method and device.**

(30) Priority : **26.05.89 NL 8901322**

(43) Date of publication of application : **28.11.90 Bulletin 90/48**

(45) Publication of the grant of the patent : **26.10.94 Bulletin 94/43**

(84) Designated Contracting States : **DE FR GB**

(56) References cited :
**EP-A- 0 193 712**
**US-A- 4 245 647**
**PROCEEDINGS OF THE 10TH SYMPOSIUM ON INFORMATION THEORY IN THE BENELUX, 25th-26th May 1989, pages 125-131, Werkgemeenschap voor Informatie- en Communicatietheorie, Enschede, NL; C.H. SLUMP: "On the prediction of the optimal exposure timing from ECG data in digital substraction angiography (DSA)"**

(56) References cited :
**MED. PHYS., vol. 10, no. 6, November/December 1983, pages 846-855, Am. Assoc.Phys. Med.; S.C. MOORE et al.: "Prospectively gated cardiac computed tomography"**
**ELECTRONICS AND COMMUNICATIONS IN JAPAN, PART I: COMMUNICATIONS, vol. 69, no.5, May 1986, pages 1-6, Scripta Technica, Inc., New York, US; K. ARAKAWA et al.: "Epsilon-Kalman Filter - A Method of Signal Estimation Considering Sudden Changes of Signals"**

(73) Proprietor : **N.V. Philips' Gloeilampenfabrieken**
**Groenewoudseweg 1**
**NL-5621 BA Eindhoven (NL)**

(72) Inventor : **Slump, Cornelis Herman**
**c/o Int. OCTROOIBUREAU B.V.,**
**Prof. Holstlaan 6**
**NL-5656 AA Eindhoven (NL)**

(74) Representative : **Zwaan, Andries Willem et al**
**INTERNATIONAAL OCTROOIBUREAU B.V.,**
**Prof. Holstlaan 6**
**NL-5656 AA Eindhoven (NL)**

EP 0 399 606 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to an X-ray exposure synchronisation method for making time-sequential X-ray images of an object that is subject to periodic movements by irradiating the object with an X-ray beam emitted by an X-ray source, the synchronisation method comprising measuring a period of movement of the object by means of a recording device, generating and applying recording pulses to a synchronisation device which activates and de-activates the X-ray source.

The invention also relates to an X-ray device for making time-sequential X-ray images of an object that is subject to periodic movements in accordance to any one of the preceding claims, the device comprising an X-ray source for emitting an X-ray beam to the object, a recording device for measuring a period of movement of the object and for generating and applying recording pulses to a synchronisation device, said synchronisation device being coupled to the X-ray source for activating and de-activating the same.

An X-ray exposure synchronisation method and a device of this kind are known from the leaflet "Philips Medical Systems", 4522 984 09241/744, DVI-V, January 1985. During the formation of an X-ray image of an object in, for example a human body, for example a heart or blood vessel, the X-ray beam is attenuated by the object and its environment. In a human body the object to be imaged and its environment consist mainly of water, so that the X-ray beam is attenuated to substantially the same extent by the object and its environment. This results in a low-contrast image of the object. By filling the object to be imaged with a contrast agent having a comparatively high X-ray absorption, such as iodide, and by subtracting an image of the object filled with contrast agent from an image of the object recorded without contrast agent, i.e. a so-called mask image, contrast enhancement is achieved. If the image of the object filled with contrast agent has been shifted with respect to the mask image due to movement of the object, artefacts will occur in the contrast-enhanced image. Notably during the formation of a series of successive X-ray images which are summed for noise integration prior to display on a television monitor, or which follow a flow of contrast agent through the object to be imaged, contrast enhancement by subtraction from a mask image will be strongly degraded by movement of the object. For the imaging of vessels situated in the vicinity of a heart, said problems will occur due to cardiac movement. By measuring a movement rhythm of an object by means of the recording device, for example the formation of an electrocardiogram, exposure can be synchronised with the movement rhythm. This takes place, for example in the Philips DVI-V system. Because the exposure is performed after a predetermined delay with respect to a recording pulse supplied by the recording device, the X-ray images are in phase and register with the mask image. This has a drawback in that in the event of fluctuations of the movement rhythm of the object to be imaged, caused, for example by the contrast agent, the X-ray images will show the object in different positions, so that artefacts will occur still when this X-ray exposure synchronisation method is used.

The invention has *inter alia* for its object to provide an X-ray exposure synchronisation method in which the occurrence of movement artefacts is reduced.

To achieve this, an X-ray exposure synchronisation method in accordance with the invention is characterized in calculating the instant of the forthcoming recording pulse from the applied recording pulses by means of arithmetic means in the synchronisation device and by determining if a recording pulse is within the regular periodic movement by comparing the calculated and actual instants of the recording pulse and in suppressing the synchronisation of the X-ray source if it is recognised, from said comparison, that a recording pulse is not within said regular periodic movement.

It is remarked that in the article by S.C. Moore *et al.* "Prospectively gated cardiac computed tomography" in Med. Phys. Vol. 10, No. 6 (1983) pages 846-855, a CT apparatus is disclosed in which the orientation of the X-ray source and detector system is adjusted with the heart rhythm of the patient, so as to obtain in the same phase of the heart cycle sufficient data for good quality image reconstruction. Adjustment is made on the basis of a prediction of the heart phase derived from a measured ECG waveform. Data acquisition is not adjusted to irregularities in the heart rhythm, but can be taken into account by operator inspection of the ECG data. As the X-ray source is always activated, data are collected in all phases of the heart cycle and the operator may manually eliminate or adjust data for the reconstruction process. The article does not indicate that, in case of irregularities, data taking can be suppressed and irradiation of the patient can be avoided if useful data cannot be acquired.

Calculation of the instant of the forthcoming recording pulse from the measured recording pulses enables exposure to take place at a constant instant in time between two successive recording pulses, that is to say in a constant object position. Variations in the movement rhythm are detected by the calculation and the synchronisation of the X-ray source is adapted to the changed movement rhythm. As the effect of recording pulses which are more distant in time is made stronger or weaker in the calculation, the synchronisation responds more quickly or less quickly to rhythm variations. When the object movement exhibits stochastic movements in addition to the systematic accelerations and decelerations, for example, additional systoles or premature ventricular contractions of a heart, the recording pulses will not succeed one another at exactly predictable instants. For accurate calculation of the forthcoming recording pulse by combination of the measured recording pulses, it is essential that the preceding pulses are correctly weighted so that not every fluctuation causes a variation of the synchronisation, without the synchronisation becoming insensitive to systematic rhythm variations. When the mean movement rhythm and the variance are characterising parameters of the stochastic process describing the objection motion, an accurate prediction of the forthcoming recording pulse can be made when the mean value and the variance are known. Usually, there is no time for an extensive statistic analysis of the object movement and a number of X-ray images must be quickly formed because of the displacement of contrast agent. The calculation should generate a value for the instant of the forthcoming recording pulse within a mean period of the recording pulses, so that it should require little calculation time. A calculation of the forthcoming recording pulse should also be fully automatic in order to simplify the formation of X-ray images.

A preferred version of an X-ray exposure synchronisation method in accordance with the invention is characterized in that a recursive Kalman filter method is used for calculating the forthcoming recording pulse.

The use of a Kalman filter method results in a recursive description of the movement rhythm, which description adapts itself to variations of this stochastic process. This calculation method adequately distinguishes non-systematic variations of the movement rhythm from systematic variations. This is advantageous notably for cardiologic angiographic examinations. A Kalman filter is known *perse*. An example of such a filter method is disclosed in the article by K. Arakawa *et al.* "ε-Kalman filter - A method of signal estimation considering sudden changes of signals", in Electronics and communications in Japan. Part I: Communications, Vol. 69, No. 5, (May 1986), pages 1-6.

The invention will be described in detail hereinafter with reference to the accompanying drawing. Therein:

Fig. 1 diagrammatically shows a device suitable for carrying out the X-ray exposure synchronisation method.

Figs. 2a to 2c show series of recording pulses with associated synchronisation pulses, and

Fig. 3 shows a flowchart of a calculation method for the forthcoming recording pulse according to the Kalman filter method.

Fig. 1 shows an X-ray source 1 which irradiates an object 3, containing a detail 4, by means of an X-ray beam 5. Using an X-ray detector 7, in this case an X-ray intensifier tube, an image carrying X-ray beam 6 is converted into an optical image which appears on an exit screen of the X-ray image intensifier tube and which is converted into an electric signal by means of a television camera tube 9. This signal is applied to an analog-to-digital converter 11 and subsequently to one of the memories 12 or 13. For example, in the memory 12 an image of the object 3 containing a detail 4 filled with contrast agent is stored and in the memory 13 a mask image of the object 3 is stored. After logarithmation of the signals in the memories 12 and 13, the digital images in the memories are subtracted in a subtraction device 15, thus producing a contrast-enhanced image of the detail 4. After digital-to-analog conversion by means of a digital-to-analog converter 16, this image is displayed on a monitor 17. The digital X-ray images stored in the memories 12 and 13 can be stored in a background memory 19 wherefrom digital images can also be applied to the memories 12 and 13. In order to determine a movement rhythm of the detail 4, a recording device 20 is connected to the detail 4, which recording device applies a pulse-shaped signal to a synchronisation device 22 at instants at which the detail 4 occupies a predetermined position. The synchronisation device 22 calculates the instant of the forthcoming recording pulse from the recording pulses and detects systematic rhythm variations of the detail 4. The synchronisation device 22 activates the X-ray source 1 in synchronism with a frame period, for example 40 ms, of a television monitor 23 displaying a real-time image of the object.

Fig. 2a shows a series of recording pulses $t_1$-$t_5$ which succeed one another with an interval T, a systematic rhythm variation occurring after the instant $t_3$. Synchronisation of the X-ray source by the synchronisation device at the instants $x_1$-$x_4$, delayed a given period of time with respect to the recording pulses, takes place at instants preceding the rhythm variation at a fraction of T which deviates from the fraction of T after the rhythm variation. By calculating the forthcoming interval T and by effecting the synchronisation at a predetermined fraction of T after the last recording pulse, the described problem is avoided. Fig. 2b shows a stochastic rhythm variation which occurs in heart rhythms: the extra systole. In that event an additional heart contraction occurs between two heart beats which succeed one another with an interval T. Synchronisation after a time delay, predetermined with respect to the last recording pulse, leads to an out-of-phase exposure at the instants $x_2$ and $x_3$. Fig. 2c shows that, if the fluctuation does not have any effect on the calculated forthcoming recording pulse, out-of-phase exposure takes place only at the instant $x_2$. By recognising the recording pulse $t_3$ as a stochastic fluctuation, the synchronisation at the instant $x_2$ can be suppressed on the basis thereof. A further fluctuation occurring in a heart rhythm is a so-called premature ventricular contraction, where one recording pulse appears earlier and where the subsequent recording pulse appears at the expected instant. In that case out-of-phase exposures occur at the instants $x_1$ and $x_2$ according to the known synchronisation method, out of phase exposure taking place exclusively at the instant $x_1$ when use is made of the synchronisation in accordance with the invention. Synchronisation at the instant $x_2$ can be suppressed after recognition of the recording pulse $t_2$ as a stochastic fluctuation.

Fig. 3 shows a flowchart for the Kalman filter method. For a mathematical description of the problem of predicting the movement rhythm of the object to be imaged, the movement rhythm is represented as a stochastic process, a three dimensional vector $\underline{x}(t)$ of which comprises the parameters relevant to the process. It has been that found that $x_1(t_n) = t_n$, $x_2(t_n) = t_n - t_{n-1}$ and $x_3(t_n) = t_n - 2t_{n-1} + t_{n-2}$ adequately characterize the process. The process is represented by the relation:

$$\underline{x}(k+1) = A\underline{x}(k) + \underline{w}(k) \quad (1)$$

Therein, the bars denote column vectors and a capital letter denotes a matrix. $\underline{w}(k)$ is represented as a stochastic white process with a mean zero:

$$E[\underline{w}(k) . \underline{w}^T(1)] = Q(k)\,\delta_{k,l} \quad (2)$$

Therein, E is the predictor operator and in $\underline{w}(k)^T$ is the transposed vector of the column vector in $\underline{w}(k)$.

The first-order autoregressive process as described in formula 1 is observed as a series of discrete measurements $\underline{y}(k)$, the recording pulses, which are related to the system by:

$$\underline{y}(k) = C\underline{x}(k) + \underline{v}(k) \quad (3)$$

Therein, C = (1,0,0) and $\underline{v}(k)$ is a white noise process disturbing the measurement values, with a mean zero and with a covariance matrix:

$$E[\underline{v}(k) . \underline{v}(l)] = R(k)\,\delta_{k,l} \quad (4)$$

Furthermore, in $\underline{w}(k)$ and $\underline{v}(k)$ are non-correlated. The problem to be solved consists in that the movement rhythm $\underline{x}(k+1)$ must be calculated from the recording pulses $y(0)...y(k)$ observed. This predictor is denoted as $\hat{\underline{x}}(k+1|k)$, where $\hat{\underline{x}}(k+1|k) = E[\underline{x}(k+1|\underline{y}(k)]$, being the conditional mean value of $\underline{x}(k+1)$ for $\underline{y}(k)$ given. Because $\underline{x}(k)$ and $\underline{y}(k)$ together have a gaussian distribution, the statistics of the movement rhythm is described fully by the conditional mean and the variance of this distribution. The common conditional probability distribution is recursively given by the Kalman filter method. The Kalman filter method minimises the mean squared error in the predicted forthcoming recording pulse $E[(t_{n+1} - t_n)^2]$. For $\hat{\underline{x}}(k+1|k)$ the Kalman filter method produces:

$$\hat{\underline{x}}(k+1|k) = A\hat{\underline{x}}(k|k-1) + G(k)[\underline{y}(k) - C\hat{\underline{x}}(k|k-1)] \quad (5)$$

or

$$\hat{\underline{x}}(k+1|k) = A\hat{\underline{x}}(k|k) \quad (5b)$$

Therein, G(k) is the predictor gain matrix. G(k) is given by:

$$G(k) = AP(k|k-1)C^T[CP(k|k-1)C^T + R(k)]^{-1} \quad (6)$$

The Kalman gain matrix K(k) is defined as:

$$AK(k) = G(k) \quad (6b)$$

P(k+1|k) is the predicted mean squared error matrix:

$$P(k+1|k) = (A - G(k)C)P(k|k-1)A^T + Q(k) \quad (7)$$

The mean squared error matrix P(k|k) is:

$$P(k|k) = E[(\hat{\underline{x}}(k) - \underline{x}(k))(\hat{\underline{x}}(k) - \underline{x}(k))^T] \quad (8)$$

For the described application:

$$A = \begin{pmatrix} 1 & 1 & 1 \\ 0 & 1 & 1 \\ 0 & 0 & 1 \end{pmatrix}$$

It is assumed that:

$$R(k) = 0.5 \text{ and } Q(k) = \begin{pmatrix} 0.5 & 0 & 0 \\ 0 & 1 & 0 \\ 0 & 0 & 2 \end{pmatrix}$$

The initialisation is:

$\hat{x}_1(3) = t_3$

$\hat{x}_2(3) = t_3 - t_2$, and

$\hat{x}_3(3) = t_3 - 2t_2 + t_1$.

For P(3|3) the initialisation is:

$$P(3|3) = \begin{pmatrix} 0.5 & 1 & 2 \\ 1 & 1 & 1 \\ 2 & 1 & 2 \end{pmatrix}$$

From (5) it follows for the predicted value of $\underline{x}$ that:

$$\hat{\underline{x}}(4|3) = A\underline{x}(3|3) = 3(t_3 - t_2) + t_1$$

and from (7) it follows for the predicted value of P that:

$$P(4|3) = AP(3|3)A^T + Q(k)$$

A correction is required at the next instant:

$$\hat{\underline{x}}(4|4) = \hat{\underline{x}}(4|3) + K(k)(\underline{y}(4) - C\underline{x}(4|3) \quad (9)$$

with K the Kalman gain matrix:

$$K(k) = P(4|3)C^T(CP(4|3)C^T + 0.5)^{-1} \quad (10)$$

P(4|4) is given by:

$$P(4|4) = P(4|3) - KCP(4|3) \quad (11)$$

After elaboration of 9, 10 and 11, $\hat{\underline{x}}(5|4)$ can be calculated by means of 5 etc.

## Claims

1. An X-ray exposure synchronisation method for making time-sequential X-ray images of an object (3) that is subject to periodic movements by irradiating the object (3) with an X-ray beam (5) emitted by an X-ray source (1), the synchronisation method comprising measuring a period of movement of the object (3) by means of a recording device (20), generating and applying recording pulses ($t_1$, $t_2$, $t_3$, ...) to a synchronisation device (22) which activates and de-activates the X-ray source (1), characterised in calculating the instant of the forthcoming recording pulse from the applied recording pulses ($t_1$, $t_2$, $t_3$, ...) by means of arithmetic means in the synchronisation device (22) and by determining if a recording pulse is within the regular periodic movement by comparing the calculated and actual instants of the recording pulse and in suppressing the synchronisation of the X-ray source if it is recognised, from said comparison, that a recording pulse is not within said regular periodic movement.

**2.** An X-ray exposure synchronisation method as claimed in Claim 1, characterised in that a recursive Kalman filter method is used for calculating the forthcoming recording pulse.

**3.** An X-ray exposure synchronisation method as claimed in Claim 2, characterised in that an object position is described as a function of time by a three-dimensional vector, elements of which are calculated from three successive recording pulses $t_n$, $t_{n-1}$ and $t_{n-2}$, a first element being equal to $t_n$, a second element being equal to $t_n$-$t_{n-1}$, and a third element being equal to $t_n$-2$t_{n-1}$+$t_{n-2}$.

**4.** An X-ray device for making time-sequential X-ray images of an object (3) that is subject to periodic movements in accordance to any one of the preceding claims, the device comprising an X-ray source (1) for emitting an X-ray beam (5) to the object (3), a recording device (20) for measuring a period of movement of the object (3) and for generating and applying recording pulses ($t_1$, $t_2$, $t_3$, ...) to a synchronisation device (22), said synchronisation device being coupled to the X-ray source (1) for activating and de-activating the same, characterised in that the synchronisation device (22) comprises arithmetic means for calculating an instant of a forthcoming recording pulse from the applied recording pulses ($t_1$, $t_2$, $t_3$, ...) and for determining if a recording pulse is within the regular periodic movement by comparing the calculated and actual instants of the recording pulse, and in that the synchronisation device is arranged to suppress synchronisation of the X-ray source if it is recognised, from said comparison, that a recording pulse is not within said regular periodic movement.

## Patentansprüche

**1.** Röntgenaufnahmesynchronisationsverfahren zum Erstellen zeitsequentieller Röntgenbilder eines Objekts (3), das periodischen Bewegungen unterliegt, durch Bestrahlung des Objekts (3) mit einem von einer Röntgenquelle (1) ausgesendeten Röntgenstrahlenbündel (5), wobei das Synchronisationsverfahren Messen einer Periode der Bewegung des Objekts (3) mittels einer Aufzeichnungseinrichtung (20), Erzeugen und Zuführen von Aufzeichnungsimpulsen ($t_1$, $t_2$, $t_3$, ...) an eine Synchronisationsanordnung (22), die die Röntgenquelle (1) aktiviert und deaktiviert, umfaßt, gekennzeichnet durch Berechnung des Zeitpunkts des zu erwartenden, nächsten Aufzeichnungsimpulses aus den zugeführten Aufzeichnungsimpulsen ($t_1$, $t_2$, $t_3$, ...) mit Hilfe von Rechenmitteln in der Synchronisationsanordnung (22) und durch Bestimmung, ob ein Aufzeichnungsimpuls innerhalb der normalen periodischen Bewegung auftritt, indem die berechneten und die tatsächlichen Zeitpunkte des Aufzeichnungsimpulses verglichen werden, und durch Unterdrückung der Synchronisation der Röntgenquelle, wenn aus dem genannten Vergleich deutlich wird, daß ein Aufzeichnungsimpuls nicht innerhalb der genannten normalen periodischen Bewegung auftritt.

**2.** Röntgenaufnahmesynchronisationsverfahren nach Anspruch 1, dadurch gekennzeichnet, daß zur Berechnung des zu erwartenden nächsten Aufzeichnungsimpulses ein rekursives Kalman-Filterverfahren verwendet wird.

**3.** Röntgenaufnahmesynchronisationsverfahren nach Anspruch 2, dadurch gekennzeichnet, daß eine Position des Objekts als Funktion der Zeit durch einen dreidimensionalen Vektor beschrieben wird, dessen Elemente aus drei aufeinanderfolgenden Aufzeichnungsimpulsen $t_n$, $t_{n-1}$, $t_{n-2}$ berechnet werden, wobei ein erstes Element gleich $t_n$, ein zweites Element gleich $t_n$-$t_{n-1}$ und ein drittes Element gleich $t_n$-2$t_{n-1}$+$t_{n-2}$ ist.

**4.** Röntgeneinrichtung zum Erstellen zeitsequentieller Röntgenbilder eines Objektes (3), das periodischen Bewegungen unterliegt, nach einem der vorhergehenden Ansprüche, mit einer Röntgenquelle (1) zum Aussenden eines Röntgenstrahlenbündels (5) in Richtung des Objekts (3), mit einer Aufzeichnungseinrichtung (20) zum Messen einer Periode der Bewegung des Objekts (3) und zum Erzeugen und Zuführen von Aufzeichnungsimpulsen ($t_1$, $t_2$, $t_3$, ...) an eine Synchronisationsanordnung (22), wobei die Synchronisationsanordnung mit der Röntgenquelle (1) gekoppelt ist, um diese zu aktivieren und zu deaktivieren, dadurch gekennzeichnet, daß die Synchronisationsanordnung (22) Rechenmittel umfaßt zur Berechnung des Zeitpunkts eines zu erwartenden, nächsten Aufzeichnungsimpulses aus den zugeführten Aufzeichnungsimpulsen ($t_1$, $t_2$, $t_3$, ...) sowie zur Bestimmung, ob ein Aufzeichnungsimpuls innerhalb der normalen periodischen Bewegung auftritt, indem die berechneten und die tatsächlichen Zeitpunkte des Aufzeichnungsimpulses verglichen werden, und daß die Synchronisationsanordnung so eingerichtet ist, daß die Synchronisation der Röntgenquelle unterdrückt wird, wenn aus dem genannten Vergleich deutlich wird, daß ein Aufzeichnungsimpuls nicht innerhalb der genannten normalen periodischen Bewegung auftritt.

**Revendications**

1. Procédé de synchronisation d'expositions aux rayons X pour la réalisation d'images de rayons X, en séquence dans le temps, d'un objet (3) qui est soumis à des mouvements périodiques par irradiation de l'objet (3) au moyen d'un faisceau de rayons X (5) émis par une source de rayons X (1), le procédé de synchronisation consistant à mesurer une période de mouvement de l'objet (3) à l'aide d'un dispositif d'enregistrement (20), à générer des impulsions d'enregistrement ($t_1$, $t_2$, $t_3$, ...) et à les appliquer à un dispositif de synchronisation (22) qui active et désactive la source de rayons X (1), caractérisé en ce que l'on calcule l'instant de l'impulsion d'enregistrement future à partir des impulsions d'enregistrement appliquées ($t_1$, $t_2$, $t_3$, ...) à l'aide de moyens arithmétiques dans le dispositif de synchronisation (22), et en déterminant si une impulsion d'enregistrement se trouve dans le mouvement périodique régulier en comparant l'instant calculé et l'instant réel de l'impulsion d'enregistrement et en ce que l'on supprime la synchronisation de la source de rayons X, si l'on constate, à partir de cette comparaison, qu'une impulsion d'enregistrement ne se trouve pas dans ledit mouvement périodique régulier.

2. Procédé de synchronisation d'expositions aux rayons X selon la revendication 1, caractérisé en ce que l'on utilise un procédé par filtre de Kalman récursif pour calculer l'impulsion d'enregistrement future.

3. Procédé de synchronisation d'expositions aux rayons X selon la revendication 2, caractérisé en ce que la position d'un objet est décrite en fonction du temps par un vecteur tridimensionnel, dont des éléments sont calculés à partir de trois impulsions d'enregistrement successives $t_n$, $t_{n-1}$ et $t_{n-2}$, un premier élément étant égal à $t_n$, un deuxième élément étant égal à $t_n$-$t_{n-1}$ et un troisième élément étant égal à $t_n$-$2t_{n-1}$+$t_{n-2}$.

4. Dispositif à rayons X pour réaliser des images de rayons X en séquence dans le temps d'un objet (3) qui est soumis à des mouvements périodiques selon l'une quelconque des revendications précédentes, le dispositif comprenant une source de rayons X (1) pour émettre un faisceau de rayons X (5) en direction de l'objet (3), un dispositif d'enregistrement (20) pour mesurer une période de mouvement de l'objet (3) et pour générer des impulsions d'enregistrement ($t_1$, $t_2$, $t_3$, ...) et les appliquer à un dispositif de synchronisation (22), ledit dispositif de synchronisation étant couplé à la source de rayons X (1) pour activer et désactiver celle-ci, caractérisé en ce que le dispositif de synchronisation (22) comprend des moyens arithmétiques pour calculer un instant d'une impulsion d'enregistrement future à partir des impulsions d'enregistrement appliquées ($t_1$, $t_2$, $t_3$, ...) et pour déterminer si une impulsion d'enregistrement se trouve dans le mouvement périodique régulier en comparant les instants calculé et réel de l'impulsion d'enregistrement, et en ce que le dispositif de synchronisation est agencé pour supprimer la synchronisation de la source de rayons X si l'on constate à partir de cette comparaison, qu'une impulsion d'enregistrement ne se trouve pas dans ledit mouvement périodique régulier.

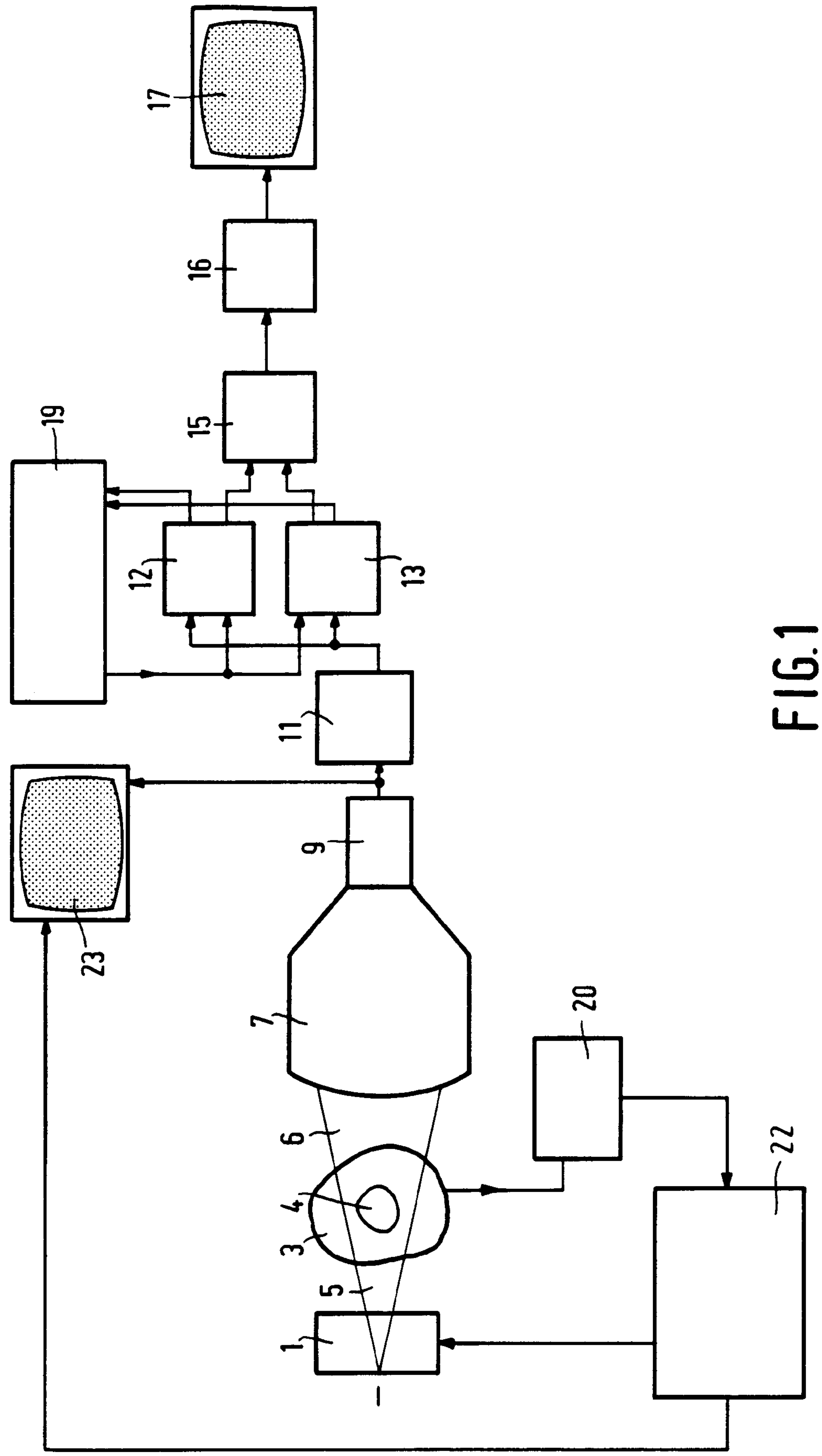


FIG.1

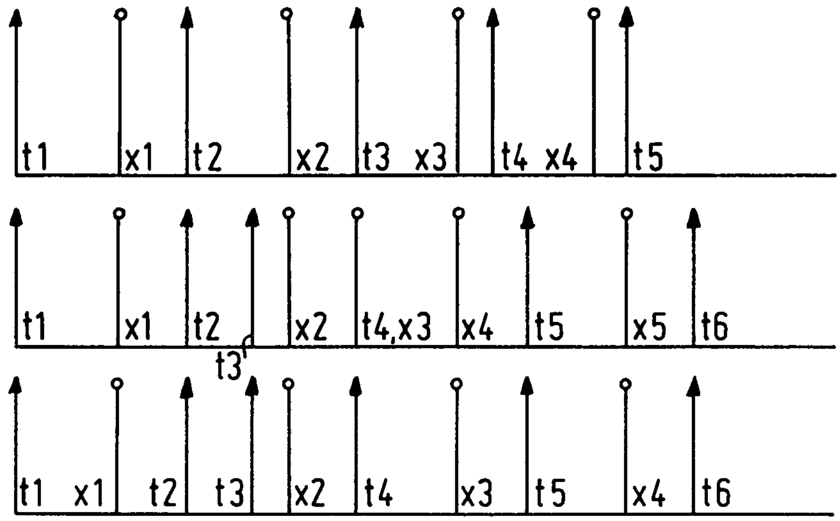


FIG.2a

FIG.2b

FIG.2c

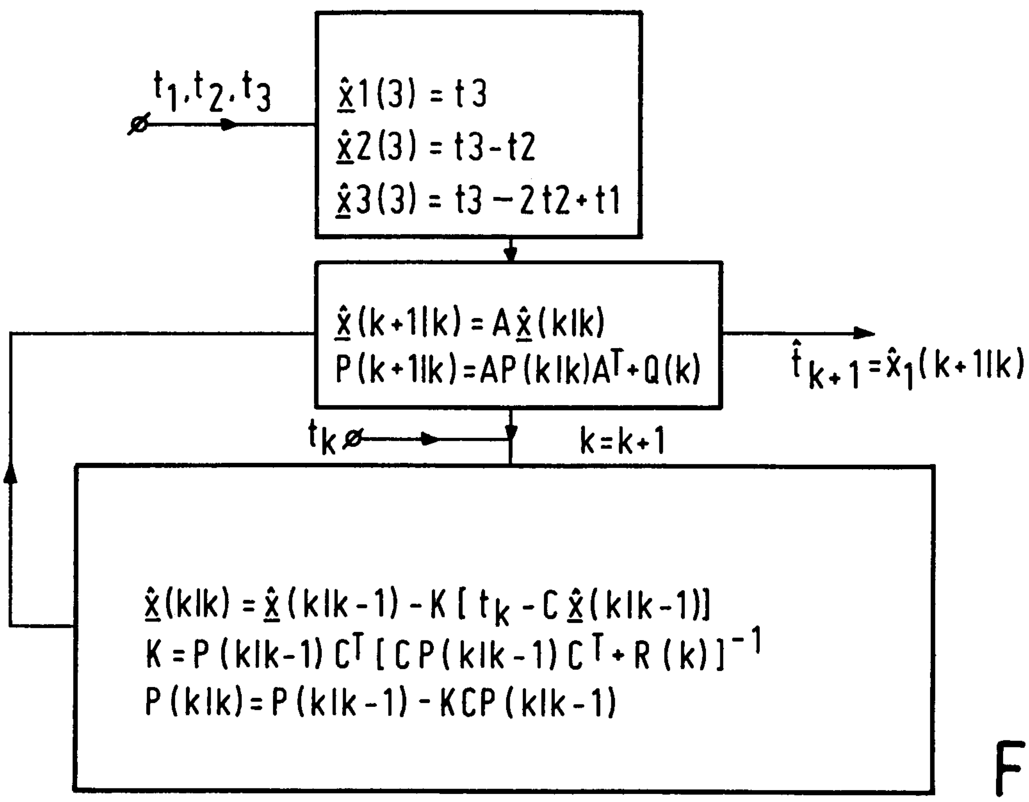


FIG. 3